# EUROPEAN PATENT APPLICATION

(11) **EP 1 693 033 A1**
(43) Date of publication of application: **23.08.2006**
(21) Application number: 04820230.3
(22) Date of filing: 08.12.2004
(51) Int. Cl.: A61F 13/472, A61F 13/514

(54) **INTER-LABIAL PAD**

(30) Priority: 09.12.2003 JP 2003411082
(71) Applicant: UNI-CHARM CORPORATION, Shikokuchuo-shi, Ehime 799-0111 (JP)
(72) Inventor: MIZUTANI, Satoshi Technical Center, Uni-Charm Corp, Mitoyo-gun, Kagawa 7691602 (JP); YOSHIMASA, Wataru Technical Center, Uni-Charm Corp, Mitoyo-gun, Kagawa 7691602 (JP); NODA, Yuki Technical Center, Uni-Charm Corporation, Mitoyo-gun, Kagawa 7691602 (JP)
(74) Representative: McNally, Roisin
(86) International application number: PCT/JP2004/018280
(87) International publication number: WO 2005/055906

(57) **Abstract**

It is an object of the present invention to provide an interlabial pad, with which even when body fluids that have been excreted out of the body reach clothes side end faces of an absorbent member, the body fluids will not leak from the interlabial pad. The present invention provides an interlabial pad, having a body fluid absorbing ability and fitted on by being sandwiched wholly or partially between labia, the interlabial pad being equipped with a liquid-impermeable barrier part, which is positioned in the direction perpendicularly downwards from the vestibular floor at the inner side between the labia and receives body fluids that flow out.

## Description

### TECHNICAL FIELD

The present invention concerns an interlabial pad to be fitted between the labia of a woman, and to be more specific, concerns an interlabial pad, having a back face side sheet that does not readily allow the permeation of body fluids.

### BACK GROUND ART

Conventionally, interlabial pads, which are fitted onto labial parts of women to absorb menstrual blood, urine, and other body fluids, have been known as absorbent articles to be used during menstruation, for protection against urinary incontinence, or for both of these purposes. Since such an interlabial pad is put in close contact with a body in comparison to a menstrual napkin and can prevent menstrual blood from dispersing and contacting the body widely, it is sanitary and clean. Also, since an interlabial pad is compact in comparison to a menstrual napkin, it is excellent and comfortable in terms of fitting sensation. An inter labial padal so provides the merit of being low in psychological reluctance during fitting in comparison to a tampon, which is inserted inside the vagina.

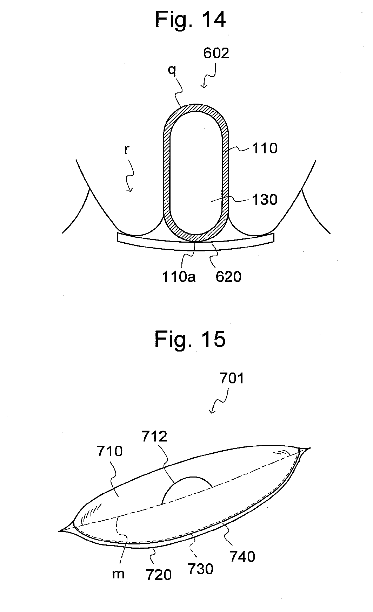

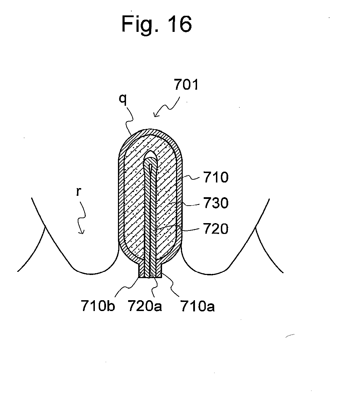

As such an interlabial pad, there is an interlabial pad (trade name: "Envive (Moderate) ") , which has been test-sold in the USA by The Procter and Gamble Corp. (referred to hereinafter as "PG Corp.") (Patent Document 1). As shown in Fig. 15, this interlabial pad 15 comprises a liquid-permeable surface side sheet 710, a liquid-impermeable back face side sheet 720, and an absorbent member 730, disposed between the surface side sheet 710 and the back face side sheet 720. Also a semicircular holding part 712, for fitting the interlabial pad 701 between the labia r, is disposed on the back face side sheet 720 of the interlabial pad 701. This interlabial pad 701 is fitted onto the labial part r upon being folded in two along a longitudinal central line m as the folding axis so that the back face side sheet 720 faces itself as shown in Fig. 16. Almost all of the clothes side end faces of the absorbent member 730 after fitting are thereby covered by the liquid-permeable surface side sheet 710. [Patent Document 1] Japanese Translation of International Application No. H-11-500341

### DISCLOSURE OF THE INVENTION

However with the interlabial pad 701, since almost all of the face of the absorbent member 730 that faces the clothes is covered by the liquid-permeable surface side sheet 710 in the fitted state, there were cases where, when body fluids, which have been excreted out of the body, reach clothes side end faces 710a, 710b, and 720a of the absorbent member 730, the body fluids permeate through the surface side sheet 710 and leak out from the interlabial pad 701.

The present invention has been made in view of the above issue, and an object thereof is to provide an interlabial pad, with which even when body fluids that have been excreted out of the body reach clothes side end faces of an absorbent member, the body fluids will not leak from the interlabial pad.

More specifically, the present invention provides the following:
(1) An interlabial pad, having a body fluid absorbing ability and being fitted on by being sandwiched wholly or partially between labia, the interlabial pad being equipped with a liquid-impermeable barrier part, which is positioned in a direction perpendicularly downwards from a vestibular floor, located at the inner side of the labia, and receives body fluids that flow out in a state in which the interlabial pad is fitted.
   In the above, "wholly or partially" means that by being sandwiched between the labia, the abovementioned interlabial pad becomes invisible as a whole from the exterior or becomes invisible in part from the exterior. "Body fluids" refer to menstrual blood, urine, vaginal discharge, etc., which are discharged from a wearer. "Body fluid absorbing ability" refers to the ability to absorb body fluids that are discharged from a wearer, and "in a direction perpendicularly downwards from the vestibular floor" refers to a part, which when the interlabial pad is fitted along with underwear or a napkin, faces the underwear or napkin in the state in which the interlabial pad is fitted. "Liquid-impermeable" refers to a property with which the permeation of body fluids is made practically difficult, and "barrier part" refers to a portion of the interlabial pad that receives body fluids discharged from a wearer.
   In the interlabial pad according to the present invention, since a portion, which is positioned in the direction perpendicularly downwards from the vestibular floor at the inner side between the labia in the fitted state, is arranged as a liquid-impermeable barrier part, body fluids, which have flowed to the barrier part of the interlabial pad from the ostium vaginae or urethral meatus, positioned at the vestibular floor, can be stopped definitely within the interlabial pad and can be held securely from leaking out of the interlabial pad. This interlabial pad may be of any shape and, for example, maybe of a flat, sheet-like form, a cylindrical form, a spherical form, a cubic form, etc.
(2) The interlabial pad according to (1), wherein the interlabial pad comprises: an absorbent member absorbing body fluids and a cover member covering the absorbent member; and wherein the barrier part is a part of the cover member.
   In the interlabial pad according to the present invention, since the absorbent member of the interlabial pad is covered by the cover member, the absorbent member that has absorbed body fluids will not contact the labia of a wearer directly so as to prevent the wearer from feeling discomfort. The wearer can thus use the interlabial pad comfortably. Also, since a part of the cover member of the present invention' s interlabial pad is made liquid-impermeable, body fluids that flow via the cover member and the absorbent member to portions of the interlabial pad opposing portions that face underwear or a napkin will be stopped by the barrier part and will be held definitely by the interlabial pad without leaking to the exterior of the interlabial pad.
(3) The interlabial pad according to (2), wherein the barrier part is detached from the inner labial walls in the fitted state.
   Since the barrier part of the interlabial pad according to the present invention does not contact the inner labial walls in the fitted state, a wearer will not feel a foreign object sensation or discomfort. The wearer can thus be fitted with the interlabial pad comfortably.
(4) The interlabial pad according to (2) or (3), wherein the interlabial pad has a practically elongate shape with a longitudinal direction and a lateral direction, and is fitted upon being folded in two along substantially the center in the longitudinal direction as the axis; wherein the cover member comprises a liquid-permeable surface side sheet, which is oriented towards a body side face, and a liquid-impermeable back face side sheet, which is oriented towards the side opposite the abovementioned body side face; wherein the back face side sheet has side edge covering parts, which extend towards the surface side sheet from respective side edges, positioned at the left and right sides with respect to the longitudinal direction as the center, and cover the respective side edges of the absorbent member; and wherein the a barrier part is formed of the side edge covering parts.
   In the above, "substantially the center in the longitudinal direction" refers to a line that extends substantially parallel to the front/back direction of the body of a wearer onto which the interlabial pad is fitted and refers to the line, which if the interlabial pad is cut along this line, the interlabial pad can be divided exactly in half longitudinally. Though if the interlabial pad is left/right symmetrical with respect to the front/back direction of a wearer, the central line will coincide with the line of symmetry, even if the interlabial pad is asymmetrical in the left/right direction, it is sufficient for the central line to be a line with respect to which the principle parts (especially the absorbent member) of the interlabial pad will be substantially symmetrical.
   The interlabial pad of the present invention is fitted upon being folded in two along substantially the center in the longitudinal direction as the axis. Since the interlabial pad can thus be put in close contact with the inner labial walls, body fluids that flow along the inner labial wall will be prevented from reaching clothes side end face of the absorbent member and the leakage of the body fluids can thus be prevented. Also, the back face side sheet of the present invention's interlabial pad has side edge covering parts, extending towards the surface side sheet from the respective side edges, which, with respect to the longitudinal direction as the central axis, are positioned at the end part sides at the respective sides (left and right sides) of the central axis, and covering the respective side edges of the absorbent member. Since the liquid-impermeable back face side sheet thereby covers the clothes face side of the interlabial pad and covers a part of the surface side sheet that covers the body side face side of the interlabial pad, the leakage of body fluids that are discharged towards portions facing underwear or a napkin can be prevented securely.
   With the interlabial pad of the present invention, in order to prevent the side edge covering parts from contacting the inner labial walls and from making a wearer feel a foreign object sensation or discomfort, the respective ends of the back face side sheet are preferably positioned at regions that are exposed from the labia.
(5) The interlabial pad according to (4), wherein the absorbent member has waist parts formed by indenting respective side parts, positioned at the left and right sides with respect to the longitudinal direction as a central axis, towards the central axis, and wherein the back face side sheet covers the absorbent member in a manner of forming spaces between itself and the waist parts.
   "Waist parts" refer to pinched parts that are provided so as to be positioned at the location of narrowest width, corresponding to the groin part, at the bases of the left and right legs of a wearer. Spaces are the parts that are provided between the back face side sheet and the waist parts of the absorbent member, and since by these spaces, menstrual blood that has reached the waist part end faces of the absorbent member is prevented from contacting the back face side sheet, better prevention of leakage of menstrual blood to a napkin or underwear can be provided.
   Since the present invention's interlabial pad has waist parts formed therein, in the fitted state, the interlabial pad will not contact the inner thighs at the groin part, positioned at the bases of the left and right legs of a wearer, and give rise to a foreign object sensation. The interlabial pad can thus be fitted onto a wearer comfortably. Also, the back face side sheet does not have the same shape as the absorbent member and spaces are formed when it covers the absorbent member. Leakage of menstrual blood can thus be prevented by these spaces. Also, when a large amount of body fluids are excreted, these spaces serve as body fluid storage spaces that can store menstrual blood. Since these spaces are covered by the liquid-impermeable back face side sheet, the stored body fluids will not leak from the absorbent member pad. Furthermore, since by the provision of these spaces, external pressures that are applied to the interlabial pad can be cushioned, a wearer will not feel a foreign object sensation.
(6) The interlabial pad according to any one of (2) to (5), wherein the cover member comprises a liquid-permeable surface side sheet, which is oriented towards a body side face, and a liquid-impermeable back face side sheet, which is oriented towards the side opposite the body side face, and wherein the barrier part comprises a bellows part, with which the surface side sheet and the back face side sheet or only the back face side sheet are or is folded towards the inner labial walls in the fitted state.
   "Bellows part" refers to a part wherein, the surface side sheet and the back face side sheet or only the back face side sheet, which are or is exposed from the labia, are or is folded so as to form one or more folds directed towards the inner labial walls.
   With the interlabial pad of the present invention, since by the barrier part comprising a bellows part, with which the surface side sheet and the back face side sheet or only the back face side sheet form or forms one or more folds directed towards the inner labial walls in the fitted state, the clothes side end faces of the back face side are prevented from contacting the inner labial walls, a wearer is made unlikely to feel a foreign object sensation or become damaged. The wearer can thus be fitted comfortably with the interlabial pad.
(7) The interlabial pad according to any one of (1) to (3), wherein the barrier part is provided so as to be substantially horizontal to a pudendal slit in the fitted state.
   "Substantially horizontal" in the above refers to the direction, which is orthogonal to the direction of discharge from the ostium vaginae or urethral meatus to portions facing underwear or a napkin in the state in which a wearer has the interlabial pad fitted, and is a direction that is parallel to the ground when the wearer stands upright.
   In the interlabial pad of the present invention, since the barrier part is provided so as to be substantially horizontal to the pudendal slit in the fitted state, the flow of body fluids can be cut off effectively.
(8) The interlabial pad according to (7), wherein the interlabial pad has an inverted T-like cross-sectional shape, formed by a flat part and a raised part, protruded from the flat part, form; and wherein the barrier part is the flat part.
   A "protrusion of inverted T-like cross-sectional shape" refers to a shape with which the barrier part, which extends in the horizontal direction, is joined to the lower end of the raised part.
   Since the barrier part of the interlabial pad of the present invention is the flat part of the protrusion of inverted T-like cross-sectional shape and is joined to the lower end of the raised part, body fluids that could not be absorbed by the absorbent member can be retained by the barrier part.
(9) The interlabial pad according to any one of (1) to (9), wherein the interlabial pad is used for a vaginal discharge absorbent member and/or for urinary incontinence.
   The abovementioned interlabial pad can be used not only for absorbing menstrual blood but can also be used for absorbing vaginal discharge or for urinary continence.
   As described above, since the present invention provides in an interlabial pad, which is fitted by the entirety or part thereof being sandwiched between the labia of a wearer and has a body fluid absorbing ability, an interlabial pad, with which the portion at the furthermost end that opposes portions of the absorbent member that face underwear or a napkin is a liquid-impermeable barrier part, an interlabial pad, with which body fluids will not leak from the interlabial pad even in the case where body fluids are excreted out of the body and reach the portion at the furthermost end, can be provided.

### BRIEF DESCRIPTION OF THE DRAWINGS

Fig. 1 is a perspective view showing an interlabial pad of a first embodiment of the present invention.
Fig. 2 is a sectional view showing the interlabial pad of the first embodiment of the present invention.
Fig. 3 is a diagram illustrating the process of manufacturing the interlabial pad of the first embodiment of the present invention.
Fig. 4 is a sectional view showing an interlabial pad of a modification example of the first embodiment of the present invention.
Fig. 5 is a sectional view showing an interlabial pad of a second embodiment of the present invention.
Fig. 6 is a sectional view showing an interlabial pad of a modification example of the second embodiment of the present invention.
Fig. 7 is a perspective view showing an interlabial pad of a third embodiment of the present invention.
Fig. 8 is a sectional view showing the interlabial pad of the third embodiment of the present invention.
Fig. 9 is a perspective view showing an interlabial pad of a fourth embodiment of the present invention.
Fig. 10 is a sectional view showing the interlabial pad of the fourth embodiment of the present invention.
Fig. 11 is a sectional view showing an interlabial pad of a fifth embodiment of the present invention.
Fig. 12 is a sectional view showing an interlabial pad of a sixth embodiment of the present invention.
Fig. 13 is a sectional view showing the interlabial pad of the seventh embodiment of the present invention.
Fig. 14 is a sectional view showing an interlabial pad of an eighth embodiment of the present invention.
Fig. 15 is a perspective view showing an interlabial pad of a prior art of the present invention.
Fig. 16 is a sectional view showing an interlabial pad of a prior art of the present invention.

### PREFERRED MODE FOR CARRYING OUT THE INVENTION

The respective embodiments of the present invention shall now be described based on the drawings. In the following description of the embodiments, the same components shall be provided with the same symbols and description thereof shall be omitted or simplified.

### First Embodiment

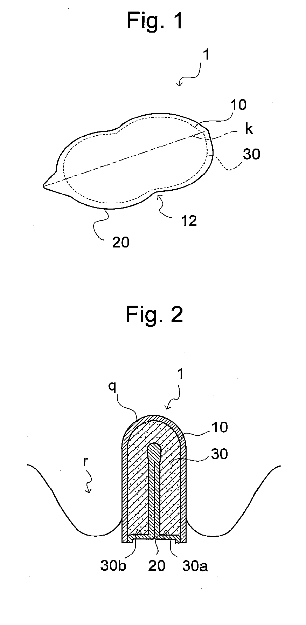

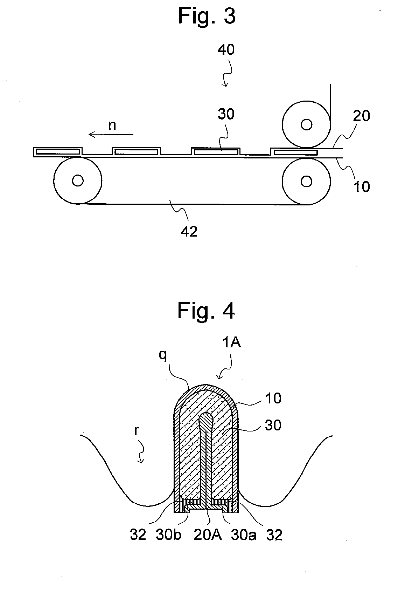

Fig. 1 is a perspective view of an interlabial pad 1 of a first embodiment of the present invention. Fig. 2 is a sectional view showing the interlabial pad 1 in the state in which it is fitted between labia. Fig. 3 is a diagram illustrating the process of manufacturing the interlabial pad 1.

The interlabial pad 1 is fitted by its entirety being sandwiched between the labia of a wearer. As shown in Fig. 1, the interlabial pad 1 is equipped with a surface side sheet 10, a back face side sheet 20, and an absorbent member 30, positioned between the surface side sheet 10 and the back face side sheet 20. The shape of the interlabial pad 1 shown in Fig. 1 is a gourd-like shape having waist parts 12, which are provided so as to be positioned at the location of the narrowest width of the groin part, positioned at the bases of the left and right legs of a wearer.

The surface side sheet 10 is a part of a covermember, contacts the body side when the inter labial pad is fitted, andhasaproperty (referred to as "liquid permeability") of allowing the permeation of body fluids, including such body fluids as menstrual blood and urine.

The back face side sheet 20 is a part of the cover member, opposes portions facing an underwear or a napkin in the fitted state, and has a property (referred to as "liquid impermeability") by which the permeation of body fluids is made practically difficult. As shown in Fig. 2, the back face side sheet 20 covers most of clothes side end faces 30a and 30b of the absorbent member 30, and the portions of the clothes side end faces 30a and 30b that are covered by the back face side sheet 20 are arranged as side edge covering parts that serve as barrier parts that receive body fluids flowing down from the vestibular floor "q".

The interlabial pad 1 is fitted on by the following procedure. As shown in Fig. 1, the interlabial pad is folded substantially in half along the center "k" in the longitudinal direction as an axis so that the back face side sheet 20 is set at the inner side and the surface side sheet 10 is set at the outer side. The folded interlabial pad 1 is then fitted with the surface side sheet 10 being the body side that contacts the body and so that the bent portion contacts the vestibular floor "q" of the labia "r" as shown in Fig. 2. With the interlabial pad 1 that has thus been fitted between the labia, since the surface side sheet 10 is liquid-permeable, body fluids that flow down the inner labial walls permeate through the surface side sheet 10 and become absorbed and held by the absorbent member 30. In this process, since the clothes side end faces 30a and 30b of the absorbent member 30 are covered by the liquid-impermeable back face side sheet 20, the body fluids that have flowed to the clothes side end faces 30a and 30b of the absorbent member 30 are stopped by the back face side sheet 20 and are held securely by the absorbent member 30 without leaking out of the interlabial pad 1.

As a method of preparing the interlabial pad, the following method can be cited. First, using an emboss roll, which models an absorbent member shape having substantially the same dimensions as the absorbent member or being 1 to 5mm larger than the absorbent member, a depression of the same shape as the absorbent member is formed in the back face side sheet. The back face side sheet may be cut out from a sheet made from the raw material for the back face side sheet or the back face side sheet may be molded by pouring resin into a mold modeling the shape of the absorbent member. The absorbent member is then filled in the depressed region of the back face side sheet that is depressed in the form of the absorbent member and then the surface side sheet is joined.

Fig. 3 illustrates the process of manufacturing the interlabial pad 1. First, a production line 40 is equipped with a suction device 42, and the surface side sheet 10 is placed on the upper face of this suction device 42. Absorbent members 30 are then placed on the surface side sheet 10 at substantially equal intervals and then the back face side sheet 20 is set on top. Thus on the upper face of the suction device 42, the surface side sheet 10, absorbent members 30, and back face side sheet 20 are layered while being conveyed in the direction of "n". Also, since the back face side sheet 20 has flexibility, the back face side sheet 20 is drawn by suction, applied across the surface side sheet 10 and the absorbent members 30, and thereby formed in compliance to the shape of the gourd-like absorbent members 30, and the side edge covering parts, which surround the edges of the absorbent member 30 and cover the clothes side end faces 30a and 30b of the absorbent member 30, are thereby formed. If in this process, the back face side sheet 20 is excellent in extensibility, the back face side sheet 20 will extend by an amount corresponding to the thickness of each absorbent member and be more readily formed in accordance to the shape of the absorbent member. The interlabial pad 1 can thereby be formed readily to the gourd-like shape. Also, the interlabial pad 1 that is manufactured has flexibility and will be unlikely to make a wearer feel a foreign object sensation in the fitted state.

Furthermore, in order for at least the clothes side end faces 30a and 30b of the absorbent member 30 to continue to be covered by the back face side sheet 20 during fitting, it is preferable for at least the clothes side end faces 30a and 30b of the absorbent member 30 to be joined to the back face side sheet 20. Specifically, an adhesive agent 32 is provided between the back face side sheet 20 and the clothes side end faces 30a and 30b of the absorbent member 30 as shown in Fig. 4.

An interlabial pad 1A of Fig. 4 is a modification example of the interlabial pad 1 of the first embodiment. With this embodiment, in place of forming the side edge covering parts by covering the clothes side end faces 30a and 30b of the absorbent member 30 by the back face side sheet 20 as in the interlabial pad 1, adhesive agent 32 is applied between the clothes side end faces 30a and 30b of the absorbent member 30 and the back face side sheet 20A to form barrier parts. The coating pattern of the adhesive agent 32 is not restricted in particular, and spiral coating, controlled seam coating, coater coating, curtain coater coating, spray gun coating, etc., may be employed.

### Second Embodiment

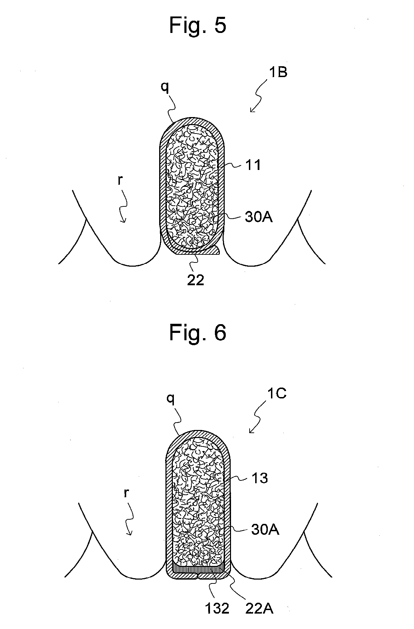

Fig. 5 is a schematic sectional view of an interlabial pad 1B with a substantially elliptical cylindrical shape. The interlabial pad 1B comprises an absorbent member 30A of substantially elliptical cylindrical shape and the surface side sheet 11, serving as the cover member that covers the absorbent member 30A, and one side part of the substantially elliptical cylindrical absorbent member 30Athat is a portion that is covered by the surface side sheet 11 is arranged as a flat part 22. The interlabial pad 1B is fitted between the labia "r" so as to put the side part at the opposite side of the flat part 22 in contact with the vestibular floor "q".

With this embodiment, the surface side sheet 11 is formed of a single sheet of liquid-permeable material, and the flat part 22 is embossed and thus made high in density, provided with liquid impermeability, and thereby arranged as the barrier part that receives body fluids flowing down from the vestibular floor "q". Though the emboss pattern may be of dot-form, wave-form, etc., and is not restricted in particular, flat embossing is preferable for increasing the liquid impermeability. The liquid-impermeable flat part 22 is not restricted to being arranged by embossing and may instead be provided by applying a lamination process or water repellency treatment, etc., to the single sheet of liquid-permeable material.

Fig. 6 shows an interlabial pad 1C, which is a modification example of interlabial pad 1B of Fig. 5. The interlabial pad 1C is an example wherein, instead of embossing a part of the surface side sheet 11 as in the interlabial pad 1B shown in Fig. 5, a flat part 22A, which serves as the barrier part, is provided by applying an adhesive agent 132 between the surface side sheet 13, which is formed of a single sheet of liquidpermeablematerial, and the absorbent member 30A. Though spiral coating, controlled seam coating, coater coating, curtain coater coating, spray gun coating, etc., can be cited as patterns of coating the adhesive agent 132, coater coating is preferable among these for increasing the liquid impermeability. Also, the method of providing the barrier part is not limited to the use of adhesive agent 132, and polyvinyl alcohol or a water repellency treatment may be applied instead to the absorbent member and/or the cover member.

### Third Embodiment

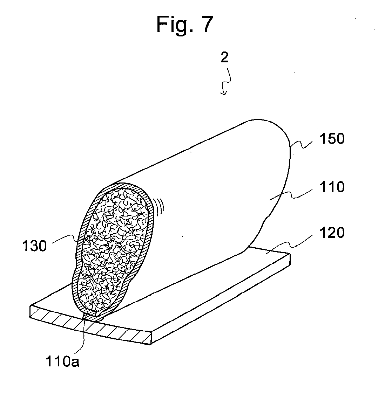

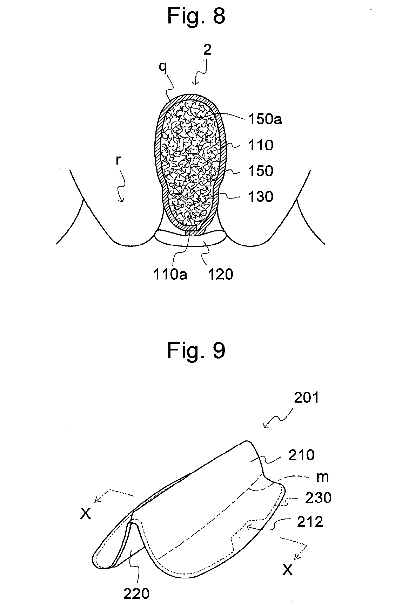

Fig. 7 is a perspective view of an end face of an interlabial pad 2 of a third embodiment of the present invention. Fig. 8 is a sectional view showing the state in which the interlabial pad 2 is fitted between the labia.

As shown in Fig. 7, the interlabial pad 2 has an inverted T-like shape with a flat part 120 and a raised part 150 and is equipped with an absorbent member 130 that is enclosed in a cover member 110. Also as shown in Fig. 8, the cross-sectional shape of the raised part 150 is an elongate, substantially elliptical shape, and the interlabial pad 2 is fitted by fitting this raised part 150 in the gap between the labia of a wearer. The width of the raised part 150 is made somewhat narrow at the flat part 120 side.

The cover member 110 has a property (referred to as "liquid permeability") of allowing the permeation of body fluids, including such body fluids as menstrual blood, urine, etc. Also, the cover member 110 has a shape that encloses the absorbent member 130.

The flat part 120 has a property (referred to as "liquid impermeability") of practically not allowing the permeation of thebodyfluids. Also, the flat part 120 is joined to and provided on the cover member 110 at a joined part 110a of the cover member 110 so as to be substantially perpendicular to the direction of outflow of body fluids that are discharged from the ostium vaginae to the portion facing underwear or a napkin.

The interlabial pad 2 is fitted on by the following procedure. First, as shown in Fig. 8, with the cover member 110 being set as the body side that comes in contact with the body, fitting is performed so as to put a curved portion 150a of the raised part 150 in contact with the vestibular floor "q" of the labia "r." Also, fitting is performed so that the flat part 120 blocks the interval between the labia "r". With the interlabial pad 2 that has thus been fitted between the labia, since the cover member 10 is liquid permeable, body fluids the flow down the inner labial walls permeate through the cover member 110 and become absorbed in the absorbent member 130. In this process, since portions opposing the portions of the absorbent member 130 that face underwear or a napkin are completely covered by the flat part 120, body fluids that have flowed to the portions opposing the portions of the absorbent member 130 that face underwear or a napkin are blocked by the flat part 120 and are kept securely in the absorbent member 130 without leaking outside the interlabial pad 2.

### Fourth Embodiment

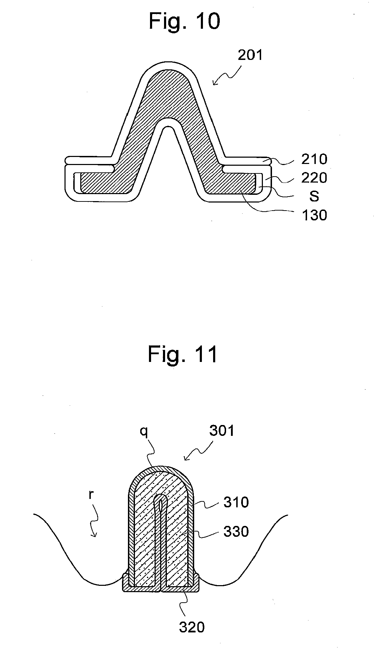

Fig. 9 is a perspective view showing an interlabial pad 201 of a fourth embodiment of the present invention. Fig. 10 is a sectional view in the X-direction of the interlabial pad 201 of the fourth embodiment of the present invention.

As shown in Fig. 9, this embodiment differs from the above-described first embodiment in that just an absorbent member 230 of the interlabial pad 201 has waist parts 212 and has a lateral-direction cross-sectional shape of a generally hill-like form with skirts. As with the interlabial pad 1 of the first embodiment, side edge cover parts are formed by a back face side sheet 220 covering clothes side end faces, including the edge parts of the absorbent member 130, in the present embodiment. As shown in Fig. 10, with the interlabial pad 201 of the present embodiment, by just the absorbent member 230 having the waist parts 212, spaces "s", which are positioned adjacent the side edge covering parts, are formed between the back face side sheet 220 and the waist parts 212. By these spaces "s", body fluids that could not be absorbed by the absorbent member 230 can be retained and these body fluids can be blocked by the back face side sheet 220. Also, even when an external pressure is applied to the interlabial pad 201, since the spaces "s" can cushion this external pressure, a wearer will not be made to feel a foreign object sensation. Also as shown in Fig. 10, the interlabial pad 201 is folded along a crease "m" so that the surface side sheet 210 faces itself. Thus even when the menstrual blood that has been absorbed by the absorbent member 230 spreads to the end parts of the absorbent member, the menstrual blood is prevented from returning towards the skin at the ends of the labia, to which pressure tends to be applied readily. Good fitting comfort can thus be provided.

### Fifth Embodiment

Fig. 11 is a sectional view showing an interlabial pad 301 of a fifth embodiment of the present invention.

As shown in Fig. 11, this embodiment differs from the above-described first embodiment in that not only the portions of an absorbent member 330 that face underwear or a napkin but a part of a surface side sheet 310 is also covered by the back face side sheet 320. Since the surface sheet 310, which is formed of a liquid-permeable material, can thus be covered by the liquid-impermeable back face side sheet 320, the leakage of body fluids can be prevented. Also, since there is no leakage of body fluids to regions of the interlabial pad 301 that are exposed from the labia "r", the interlabial pad 301 can be removed without worry of soiling of a finger.

### Sixth Embodiment

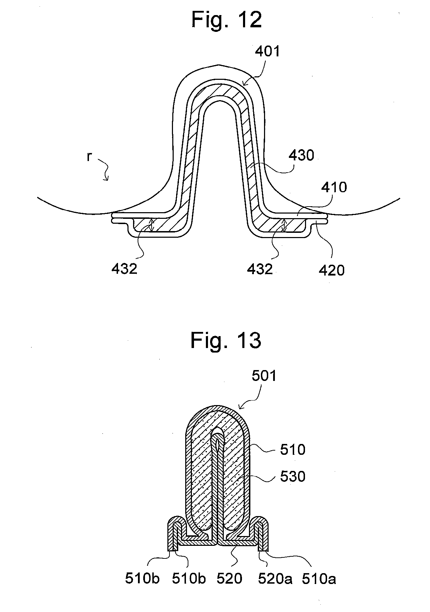

Fig. 12 is a sectional view of an interlabial pad 401 of a sixth embodiment of the present invention.

As shown in Fig. 12, this embodiment differs from the above-described first embodiment in that a surface side sheet 410 and a back face side sheet 420 of the interlabial pad 401 are formed so as to be exposed from the labia "r" and set along the labia "r". Since, as shown in Fig. 12, the surface side sheet 410 is thus kept in a substantially horizontal state without bending in the thickness direction 432 of an absorbent member 430 and both ends of the surface side sheet 410 and the back face side sheet 420 can be kept in the state of being in contact with and not separating from the labia "r", even when body fluids flow out of the body from and along the labia "r", the body fluids can be blocked. Since the same applies not only to both ends of the surface side sheet 410 and the back face side sheet 420 but also to the front and rear end parts, side edge covering parts, which are to serve as the barrier parts, are most preferably provided at peripheral parts of the absorbent member 430 as shown in the figure. Even in the case where the peripheral parts of the surface side sheet 410 and the back face side sheet 420 are fitted inside the labia "r", the menstrual blood that has flowed along the inner walls of the labia "r" can likewise be blocked readily.

### Seventh Embodiment

Fig. 13 is a sectional view showing the interlabial pad 501 of a seventh embodiment of the present invention.

As shown in Fig. 13, this embodiment differs from the above-described first embodiment in that a surface side sheet 510 and a back face side sheet 520 of the interlabial pad 501 comprise bellows parts, which are exposed from the labia "r" and are folded once each towards the inner labial walls in the fitted state so as to be set along the labia "r". Since the respective end faces 510a, 510b, 520a, and 520b of the surface side sheet 510 and the back face side sheet 520 are thus prevented from contacting the inner labial walls as shown in Fig. 13, a wearer will not be subj ect to a foreign obj ect sensation or damage. Also, just the back face side sheet 520 of the interlabial pad 501 may comprise bellows parts that are folded towards the inner labial walls in the fitted state.

### Eighth Embodiment

Fig. 14 is a sectional view showing an interlabial pad 602 of an eighth embodiment of the present invention.

As shown in Fig. 14, this embodiment differs from the above-described second embodiment in that a flat part 620 of the interlabial pad 602 is formed so as to cover the ends of the labia "r". Since the flat part 620 thus covers the ends of the labia "r" as shown in Fig. 14 and can be kept in the state of contacting and not separating from the ends of the labia "r", even when body fluids flow along and out of the body from the ostium vaginae, the body fluids can be blocked.

The present invention is not limited to the above-described embodiments, and modifications, improvements, etc., within a scope in which the object of the present invention can be achieved, are included within the present invention. For example, though the shape of the interlabial pad 1 of the above-described embodiments is a gourd-like shape provided with pinched parts so that it can be positioned at the location of narrowest width of the groin part, positioned at the bases of the left and right legs of a wearer, the present invention is not limited thereto, and the shape of the interlabial pad is not restricted in particular and may be an elliptical shape without pinched parts, a teardrop shape, or other shape as long as it is a shape that fits the labia of women and changes in shape in the fitted state. Also, though the interlabial pad 1 of the above-described embodiments is made three-dimensional by the width-direction cross-sectional shape being bent in a substantially V-like manner, the interlabial pad may be substantially flat instead.

The present invention's interlabial pads are prepared in reference to average dimensions of women in their twenties to fifties. Specifically, the interlabial pads were prepared based on a BMI value of 21 (= weight kg / (height m)²) and total distances, between opposing groin parts in an erect posture with the inner interval between knees being 35cm, of 60mm at the anterior labial commissure, 38mm at the clitoris, 34mm at the ostium vaginae, and 50mm at the anus. These values were determined by drawing a horizontal line at each part (anterior labial commissure, clitoris, etc.,) in the longitudinal direction of the body and measuring the total distance between the deepest points of the groin that intersect with the horizontal line.

### Component Materials of the Interlabial Pad

### <Surface Side Sheet and Cover Member>

For the surface side sheets 10, 210, 310, 410, and 510 and cover member 110, which are positioned at the body sides of the respective interlabial pads, a raw material, which is water-permeable, liquid-compatible, and does not irritate the skin, is used.

A single type of non-woven fabric obtained by melt blowing, spunbonding, pointbonding, throughairmethod, needlepunching, dry- or wet-form spun lacing, foam film method, or other manufacturing method, or a composite material of such fabrics can be cited as examples of such a raw material. Among such materials, an arrangement, having at least cellulose-based, liquid-compatible fibers as the principle component, is preferable in consideration of the compatibility with the inner labial walls so that a wearer will not feel a foreign object sensation due to deviation of the interlabial pad with respect to the labial surface. Specifically, a spun-lace non-woven fabric, prepared by mixing 5 to 30% natural cotton with 70 to 95% rayon or acetate, adjusting the basis weight of the mixed fibers to within the range of 20 to 50g/m², entangling the fibers with each other by hydroentanglement, and then drying and adjusting the thickness to within the range of 0.3 to 1.0mm, is preferable. In regard to the quality of the fibers to be used, natural cotton of a fiber length in the range of 15 to 60mm and rayon or acetate of a fiber length in the range of 25 to 51mm and a fineness in the range of 2.2 to 6.6dtex are selected.

### <Back Face Side Sheet and Barrier Part>

For the liquid-impermeable back face side sheets 20, 220, 320, 420, and 520, which are used in the respective interlabial pads, a water-impermeable raw material is used. As the material of this water-impermeable sheet, a sheet, which can prevent the menstrual blood, held by the absorbent member, from leaking out of the interlabial pad, is used. Also, by using a moisture-permeable material, mustiness during fitting can be alleviated and discomfort in the fitted state can thus be lightened.

Examples of a material for the water-impermeable sheet include polyethylene, polypropylene, polyethylene terephthalate, polyvinyl alcohol, polylactic acid, polybutyl succinate, non-woven fabric, paper, and laminates of such materials with a thickness of 15 to 60µm. An air-permeable film, obtained by filling with an inorganic filler and performing a drawing process, may also be used. A film, having low-density polyethylene (LDPE) resin as the principal component, having open pores of a pore diameter of 0.1 to 0.6mm at a porosity of 10 to 30%, and being adjusted in basis weight per unit area to within the range of 15 to 35g/m², can be cited as a specific example. Spun-bonded non-woven fabrics, point-bonded non-woven fabrics, through-air non-woven fabrics, etc., can be cited as examples of non-woven fabrics and these may be subject to a water-repellent treatment. Among these, a non-woven fabric with a three-layer, spun-bonded/melt-blown/spun-bonded (SMS) arrangement, which is formed of ultrafine fibers and includes a melt-blown layer of extremely small interfiber distance, is preferable. In this case, the layers are preferably arranged to have basis weights in the ranges of 5 to 15 g/mm², 1 to 10 g/mm², and 5 to 10 g/mm², respectively.

### <Absorbent Member>

As each of the absorbent members 30, 130, 230, 330, 430, and530, any absorbent member may be used with out any restrictions in particular as long as it can absorb and hold body fluids. As each of the absorbent members 30, 130, 230, 330, 430, and 530, it is preferable to use a highly absorbent type absorbent member, which is high in the property of absorbing body fluids and, for example, can absorb and hold an amount of body fluids of approximately 15 to 100 times its own weight, is preferable, and it is furthermore preferable to use an absorbent member that is bulky, lasting in form, and low in chemical irritability. As the material for forming the absorbent members 30, 130, 230, 330, 430, and 530, pulp, chemical pulp, rayon, acetate, natural cotton, polymer absorbent member, fibrous polymer absorbent member, or synthetic fibers, etc. , may be used in solitary form or as a mixture. Carboxymethylcellulose (CMC) fibers, which are biodegradable, are especially preferable. The shapes of the absorbent members 30, 130, 230, 330, 430, and 530 are not restricted in particular and may be sheet-like or granular.

The present invention can be used as an interlabial pad that does not readily leak body fluids.

## Claims

1. An interlabial pad, having a body fluid absorbing ability and being fitted on by being sandwiched wholly or partially between labia, the interlabial pad being equipped with a liquid-impermeable barrier part, which is positioned in a direction perpendicularly downwards from a vestibular floor, located at the inner side of the labia, and receives body fluids that flow out in a state in which the interlabial pad is fitted.

2. The interlabial pad according to claim 1, wherein the interlabial pad comprises: an absorbent member absorbing body fluids and a cover member covering the absorbent member; and wherein
the barrier part is a part of the cover member.

3. The interlabial pad according to claim 2, wherein the barrier part is detached from the inner labial walls in the fitted state.

4. The interlabial pad according to claim 2 or 3, wherein the interlabial pad has a practically elongate shape with a longitudinal direction and a lateral direction, and is fitted upon being folded in two along substantially the center in the longitudinal direction as the axis; wherein
the cover member comprises a liquid-permeable surface side sheet, which is oriented towards a body side face, and a liquid-impermeable back face side sheet, which is oriented towards the side opposite the abovementioned body side face; wherein
the back face side sheet has side edge covering parts, which extend towards the surface side sheet from respective side edges, positioned at the left and right sides with respect to the longitudinal direction as the center, and cover the respective side edges of the absorbent member; and wherein
the barrier part is formed of the side edge covering parts.

5. The interlabial pad according to claim 4, wherein the absorbent member has waist parts formed by indenting respective side parts, positioned at the left and right sides with respect to the longitudinal direction as a central axis, towards the central axis, and wherein
the back face side sheet covers the absorbent member in a manner of forming spaces between itself and the waist parts.

6. The interlabial pad according to any one of claims 2 to 5, wherein the cover member comprises a liquid-permeable surface side sheet, which is oriented towards a body side face, and a liquid-impermeable back face side sheet, which is oriented towards the side opposite the body side face, and wherein
the barrier part comprises a bellows part, with which the surface side sheet and the back face side sheet or only the back face side sheet are or is folded towards the inner labial walls in the fitted state.

7. The interlabial pad according to any one of claims 1 to 3, wherein the barrier part is provided so as to be substantially horizontal to a pudendal slit in the fitted state.

8. The interlabial pad according to claim 7, wherein the interlabial pad has an inverted T-like cross-sectional shape, formed by a flat part and a raised part, protruded from the flat part, form; and wherein
the barrier part is the flat part.

9. The interlabial pad according to any one of claims 1 to 9, wherein the interlabial pad is used for a vaginal discharge absorbent member and/or for urinary incontinence.
